# EUROPÄISCHE PATENTSCHRIFT

(11) **EP 3 507 270 B1**
(45) Veröffentlichungstag und Bekanntmachung des Hinweises auf die Patenterteilung: **02.12.2020**
(21) Anmeldenummer: 16760071.7
(22) Anmeldetag: 02.09.2016
(51) Int. Cl.: C07C 68/02, C07C 69/96

(54) **VERFAHREN ZUR HERSTELLUNG VON DIALKYLDICARBONATEN UNTER VERWENDUNG TERTIÄRER AMINE ALS KATALYSATOREN**
METHOD FOR PRODUCING DIALKYLDICARBONATES USING TERTIARY AMINES AS CATALYSTS
PROCÉDÉ DE FABRICATION DE DIALKYLDICARBONATES AU MOYEN D'AMINES TERTIAIRES UTILISÉES COMME CATALYSEURS

(43) Veröffentlichungstag der Anmeldung: 10.07.2019
(73) Patentinhaber: Lanxess Deutschland GmbH, 50569 Köln (DE)
(72) Erfinder: VOGL, Erasmus, 51429 Bergisch-Gladbach (DE); HOFMANN, Christoph, 51061 Köln (DE)
(86) Internationale Anmeldenummer: PCT/EP2016/070701
(87) Internationale Veröffentlichungsnummer: WO 2018/041360

(56) Entgegenhaltungen:
- WO-A1-2005/110964
- DE-B- 1 210 853
- US-A- 5 231 211

## Beschreibung

Die vorliegende Erfindung betrifft ein Verfahren zur Herstellung von Dialkyldicarbonaten aus den entsprechenden Chlorameisensäurealkylestern unter Verwendung spezieller tertiärer Amine als Katalysatoren.

Dialkyldicarbonate finden z.B. als Katalysatoren zur Oxidation von sterisch anspruchsvollen Aminen, als Bestandteile von Elektrolytflüssigkeiten oder als Bestandteile von antimikrobiellen Reagenzien Verwendung. Dialkyldicarbonate werden in der Literatur auch als Dialkylpyrocarbonate bezeichnet.

Aus DE 1 210 853 B ist bekannt, Kohlensäure- oder Carbonsäurehalogenide mit organischen Hydroxylverbindungen oder deren Alkali- oder Erdalkalisalzen und organischen, mit Wasser nicht mischbaren Lösungsmitteln und mindestens äquivalenten Mengen an Alkali- oder Erdalkalihydroxiden oder -carbonaten in einem zweiphasigen System, sowie unter Verwendung katalytischer Mengen an tertiären Aminen oder ihren Quaternierungsprodukten, umzusetzen, wobei als Amine oder deren Quaternierungsprodukte solche eingesetzt werden, die mindestens eine an Stickstoff gebundene ω-Hydroxyalkyl-, ω-Hydroxyalkyläther- oder ω-Hydroxyalkylpolyäthergruppe tragen.

In DE-A 1 418 849 werden weiterhin tertiäre Amine als besonders geeignete Katalysatoren für die Herstellung von Säurederivaten beschrieben, deren tertiäre Stickstoffatome sterisch nicht gehindert sind, ausgenommen tertiäre Amine, die als Substituenten am Stickstoff ebensolche ω-Hydroxyalkyl-, ω-Hydroxyalkyläther- oder ω-Hydroxyalkylpolyäthergruppe tragen. Neben Triethylamin und Tri-n-butylamin kommen hier deshalb Amine zum Einsatz, die mindestens eine Methylgruppe am Stickstoff tragen, wie z.B. N-Methyl-di-n-stearylamin. Diese Katalysatoren besitzen aber u.a. den Nachteil, dass sie nicht nur die Bildung, sondern auch die Zersetzung des Produktes katalysieren, was zu einer Verringerung der Ausbeute führt. Zudem sind einige dieser Katalysatoren toxisch, werden im Abwasser schlecht abgebaut und lassen sich vom Reaktionsgemisch aufgrund eigener Zersetzungen während der Reaktion schlecht abtrennen.

Aus der EP 1747185 A ist ein Verfahren zur Herstellung von Dialkyldicarbonaten aus Halogenameisensäurealkylestern durch Umsetzung mit Alkali- oder Erdalkalihydroxiden oder - carbonaten bekannt, bei dem langkettige tertiäre C₆-C₂₅-Alkylamine eingesetzt werden. Auch diese Katalysatoren sind toxisch, zersetzen sich im Abwasser schlecht und stellen daher eine ökologische Belastung dar.

Es bestand daher Bedarf an einem Herstellungsverfahren, welches das Zielprodukt in hoher Ausbeute liefert und bei dem die Katalysatoren besser abgetrennt werden können.

Überraschenderweise wurde gefunden, dass Dialkyldicarbonate aus Halogenameisensäurealkylestern durch Umsetzung mit Alkali- oder Erdalkalihydroxiden oder -carbonaten besonders vorteilhaft erhalten werden können, wenn man als Katalysator spezielle langkettige tertiäre Amine der Formel (I) einsetzt. Diese zeichnen sich durch eine hohe katalytische Aktivität aus, ohne zersetzend auf das Endprodukt zu wirken und können leicht vom Produkt, z.B. destillativ, abgetrennt werden. Zudem sind sie weniger toxisch als vergleichbare PhasenTransferkatalysatoren und werden im Rahmen der Abwasserbehandlung besser abgebaut.

Gegenstand der vorliegenden Erfindung ist demnach ein Verfahren zur Herstellung von Dialkyldicarbonaten durch Umsetzung der entsprechenden Halogenameisensäurealkylester mit Alkali- oder Erdalkalihydroxiden und/oder -carbonaten in Gegenwart von mit Wasser nicht mischbaren organischen Lösungsmitteln und in Gegenwart eines Katalysators dadurch gekennzeichnet ist, dass als Katalysator mindestens ein tertiäres Amin der Formel (I)

NR¹R²R³ (I)

worin
R¹ = geradkettiges oder verzweigtes C₁-C₆-Alkyl,
R² = geradkettiges oder verzweigtes C₁₆-C₂₂-Alkyl und
R³ = -[CH₂-R⁴-O]ₙ-[CH₂-R⁴]-OH ist, wobei n = 0 bis 12 und R⁴ = -[CH₂-CH₂]-, -[CH(CH₃)]- oder -CH₂- ist,
eingesetzt wird.

Bevorzugt werden bei der Durchführung des erfindungsgemäßen Verfahrens als Katalysator tertiäre Amine der Formel (I) eingesetzt, worin
R¹ = Methyl, Ethyl, Propyl oder Butyl ist,
R² = geradkettiges oder verzweigtes C₁₇-C₂₀-Alkyl und
R³ = -[CH₂-(CH(CH₃))-O]ₙ-[CH₂-(CH(CH₃))]-OH, -[CH₂-CH₂-CH₂-O]ₙ-[CH₂-CH₂-CH₂]-OH oder -[CH₂-CH₂-O]ₙ-[CH₂-CH₂]-OH mit n = 6 bis 12 stehen.

Besonders bevorzugt wird der Katalysator mit R¹ = Methyl, R²= geradkettiges C₁₈-Alkyl und R³ = - [CH₂-CH(CH₃)-O]ₙ-[CH₂-CH(CH₃)]-OH mit n = 8 eingesetzt.

In einer Ausführungsform des erfindungsgemäßen Verfahrens wird ein Amin der allgemeinen Formel (I) als Katalysator eingesetzt, bei dem R² jeweils geradkettiges oder verzweigtes Hexadecanyl, Heptadecanyl, Octadecanyl, Nonadecanyl, Eicosanyl, Icosanyl, Heneicosanyl oder Dodoconyl bedeutet. Bevorzugt ist R² = geradkettiges Octadecanyl.

Bei der Durchführung des erfindungsgemäßen Verfahrens können auch beliebige Mischungen der Katalysatoren eingesetzt werden. In einer bevorzugten Ausführungsform des erfindungsgemäßen Verfahrens werden als Katalysator Mischungen von zwei oder mehr tertiären Aminen der Formel (I) eingesetzt, bei denen R³ für -[CH₂-(CH(CH₃))-O]ₙ-[CH₂-(CH(CH₃))]-OH steht und n verschiedene Werte im Bereich von 8 bis 11 besitzt, besonders bevorzugt = 8, 9, 10 oder 11 ist.

In einer weiteren bevorzugten Ausführungsform werden als Katalysatoren Mischungen von Verbindungen der Formel (I) eingesetzt, die verschiedene Reste R² mit geradkettigem oder verzweigtem C₁₆-, C₁₇-, C₁₈-, C₁₉-, C₂₀- oder C₂₁-Alkyl aufweisen.

In einer weiteren bevorzugten Ausführungsform können als Katalysatoren Mischungen der Verbindungen der Formel (I) eingesetzt werden, bei denen die Reste R¹, R² und R³ im Rahmen der obigen Offenbarung in beliebiger Art kombiniert werden.

Die zur Herstellung der Katalysatoren üblicherweise verwendeten Dialkylamine, wie beispielsweise N-Methylstearylamin, sind kommerziell erhältlich. Ihre Herstellungsverfahren sind ferner dem Fachmann bekannt. Auch die Herstellung der speziellen tertiären Amine der Formel (I) ist beispielsweise aus der DE 1210853 B bekannt und erfolgt üblicherweise durch Umsetzung der Dialkylamine mit Propylenoxid oder Äthylenoxid in der Gegenwart von Alkalihydroxiden.

Vorzugsweise dient das erfindungsgemäße Verfahren zur Herstellung von Dialkyldicarbonaten der Formel (II) 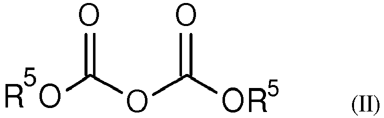 worin
R⁵ für geradkettiges oder verzweigtes C₁-C₂₀-Alkyl steht,
durch Umsetzung von Halogenameisensäurealkylester der Formel (III) 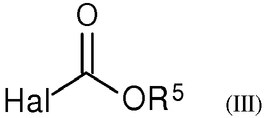 worin
Hal für Halogen, bevorzugt für F, Cl, Br, I, insbesondere für Chlor steht, und
R⁵ für geradkettiges oder verzweigtes C₁-C₂₀-Alkyl steht,
dadurch gekennzeichnet, dass die Umsetzung in Gegenwart mindestens eines tertiären Amins der Formel (I)

   NR¹R²R³ (I)

   worin
   R¹ = geradkettiges oder verzweigtes C₁-C₆-Alkyl
   R² = geradkettiges oder verzweigtes C₁₆-C₂₂-Alkyl und
   R³ = -[CH₂-R⁴-O]ₙ-[CH₂-R⁴]-OH ist, wobei n = 0 bis 12 und R⁴ = -[CH₂-CH_{2]}-, -[CH(CH₃)]- oder -CH₂- ist,
   als Katalysator durchgeführt wird.

In den Formeln (II) und (III) steht R⁵ vorzugsweise für geradkettiges oder verzweigtes C₁-C₈-Alkyl, besonders bevorzugt für einen Rest -CH-R⁶R⁷, wobei R⁶ und R⁷ unabhängig voneinander für H oder für geradkettiges oder verzweigtes C₁-C₇-Alkyl stehen. Insbesondere steht R⁵ für Methyl, Ethyl, n-Propyl, iso-Propyl, n-Butyl oder i-Butyl. Insbesondere bevorzugt steht R⁵ für Methyl, so dass Dimethyldicarbonat als Verbindung der Formel (II) erhalten wird.

Als Alkali- oder Erdalkalihydroxide oder -carbonate kommen beispielsweise LiOH, NaOH, KOH, LiCO₃, Na₂CO₃, K₂CO₃ in Frage. Vorzugsweise werden Alkalihydroxide verwendet, wie Natrium- und Kaliumhydroxid, die vorzugsweise in Form von wässrigen Lösungen zum Einsatz gelangen. Beispielsweise kann man 1 bis 50 gew.-%ige wässrige Alkalihydroxidlösungen verwenden. Bevorzugt sind 5 bis 35 gew.-%ige Lösungen, besonders bevorzugt 10 bis 25 gew.%ige Lösungen. Die Alkali- oder Erdalkalihydroxide oder -carbonate kann man beispielsweise in Mengen von 80 bis 120 Mol-% bezogen auf eingesetzten Halogenameisensäureester einsetzen. Vorzugsweise liegt diese Menge im Bereich von 90 bis 110 Mol-%, besonders bevorzugt im Bereich von 95 bis 105 Mol-%.

Als mit Wasser nicht mischbare organische Lösungsmittel kommen beispielsweise aliphatische und aromatische Kohlenwasserstoffe, halogenierte Kohlenwasserstoffe, mit Wasser nicht mischbare Ether oder Ester sowie Dialkylcarbonate in Frage. Bevorzugt sind Cyclohexan, Toluol, Xylol, Methylenchlorid und Diethylether, insbesondere Toluol und Methylenchlorid.

Das mit Wasser nicht mischbare organische Lösungsmittel kann man beispielsweise in Mengen von 20 bis 90 Gew.-%, bevorzugt von 30 bis 80 Gew.-%, besonders bevorzugt von 40 bis 70 Gew.-% bezogen auf den Halogenameisensäureester der Formel (I) einsetzen.

Der Katalysator der Formel (I) wird im Allgemeinen bezogen auf Halogenameisensäureester, in einer Menge von 0,001 bis 0,5 Mol-%, vorzugsweise von 0,005 bis 0,05 Mol-% eingesetzt.

Das erfindungsgemäße Verfahren kann in einem Druckbereich von 1 bis 10 bar, vorzugsweise von 1 bis 1,5 bar durchgeführt werden.

Die Reaktionstemperatur kann beispielsweise zwischen -10°C und der Siedetemperatur (bei Normaldruck) des eingesetzten Halogenameisensäureesters liegen. Vorzugsweise liegt sie im Bereich 0 bis 50°C.

Es ist vorteilhaft, während der Durchführung des erfindungsgemäßen Verfahrens für eine gute Durchmischung zu sorgen, z.B. durch Einsatz von Rührwerken, Strömungsstörern oder Umwälzpumpen.

Das erfindungsgemäße Verfahren kann sowohl batchweise als auch kontinuierlich durchgeführt werden. Bei batchweiser Arbeitsweise wird die Umsetzung bevorzugt in einem Rührkessel durchgeführt. Die Reaktion ist hierbei je nach Größe des Ansatzes und der vorhandenen Kühlleistung im allgemeinen nach 10 Minuten bis 3 Stunden beendet.

Vorzugsweise wird das erfindungsgemäße Verfahren kontinuierlich unter Verwendung eines Rührkessels, einer Rührkesselkaskade oder eines Rohrreaktors durchgeführt. Hierbei beträgt die mittlere Verweilzeit im Reaktor in der Regel zwischen 1 und 60 Minuten, bevorzugt zwischen 6 und 45 Minuten und besonders bevorzugt zwischen 10 und 20 Minuten.

Nach der Durchführung des erfindungsgemäßen Verfahrens, gegebenenfalls nach Abkühlung trennt sich das Reaktionsgemisch in zwei Phasen auf. Die organische Phase enthält neben dem Lösungsmittel das hergestellte Dialkyldicarbonat und gegebenenfalls geringe Mengen nicht umgesetzten Halogenameisensäureester sowie den Katalysator. Die wässrige Phase enthält neben Wasser die gebildeten anorganischen Salze.

Aus der organischen Phase kann man das Reaktionsprodukt durch mehrstufige Destillation in hoher Reinheit gewinnen. Der Katalysator kann dabei als Schwersieder abgetrennt werden und gegebenenfalls nach einer Aufreinigung wieder in dem erfindungsgemäßen Verfahren als Katalysator eingesetzt werden (Recyclisierung).

Es ist ein besonderer und überraschender Vorteil des erfindungsgemäßen Verfahrens, dass der Katalysator die Zersetzung der Dialkyldicarbonate nach der Reaktion praktisch nicht katalysiert und daher durch Destillation abgetrennt werden kann, wodurch die isolierte Ausbeute an Endprodukt im Vergleich zu herkömmlichen Verfahren höher ist. Die eingesetzten Katalysatoren sind den bisherigen bekannten Phasen-Transfer Katalysatoren zudem im Hinblick auf Abtrennung und Rückgewinnung deutlich überlegen. Auch die bessere Abbaubarkeit gegebenenfalls im Abwasser enthaltener Katalysatoren der Formel (I) im Rahmen der Abwasserbehandlung ist ein entscheidender Vorteil gegenüber den Katalysatoren des Standes der Technik.

## Patentansprüche

1. Verfahren zur Herstellung von Dialkyldicarbonaten durch Umsetzung der entsprechenden Halogenameisensäurealkylester mit mindestens einem Alkalihydroxid, Erdalkalihydroxid und/oder -carbonat in Gegenwart von mindestens einem, mit Wasser nicht mischbaren organischen Lösungsmittel und in Gegenwart eines Katalysators **dadurch gekennzeichnet, dass** als Katalysator mindestens ein tertiäres Amin der Formel (I)
NR¹R²R³ (I)
worin
R¹ = geradkettiges oder verzweigtes C₁-C₆-Alkyl ist,
R² = geradkettiges oder verzweigtes C₁₆-C₂₂-Alkyl und
R³ = -[CH₂-R⁴-O]ₙ-[CH₂-R⁴]-OH ist, wobei n = 0 bis 12 und R⁴ = -[CH₂-CH_{2]}-, -[CH(CH₃)]- oder -CH₂- ist,
eingesetzt wird.

2. Verfahren gemäß Anspruch 1, **dadurch gekennzeichnet dass** als Katalysator mindestens ein tertiäres Alkylamin der Formel (I) gemäß Anspruch 1 eingesetzt wird, worin R¹= Methyl, Ethyl, Propyl oder Butyl ist und R² = C₁₇-C₂₀-Alkyl und R³ = -[CH₂-CH(CH₃)-O]ₙ-[CH₂-CH(CH₃)]-OH oder -[CH₂-CH₂-CH₂-O]ₙ-[CH₂-CH₂-CH₂]-OH mit n = 6 bis 10 stehen.

3. Verfahren gemäß Anspruch 1 oder 2, **dadurch gekennzeichnet dass** als Katalysator mindestens ein tertiäres Amin der Formel (I) gemäß Anspruch 1 eingesetzt wird, worin R¹ = Methyl, R² = geradkettiges C₁₈-Alkyl und R³ = -[CH₂-CH(CH₃)-O]ₙ-[CH₂-CH(CH₃)]-OH mit n = 8 eingesetzt.

4. Verfahren gemäß wenigstens einem der Ansprüche 1 bis 3, **dadurch gekennzeichnet dass** als Katalysator eine Mischung von zwei oder mehr tertiären Aminen der Formel (I) eingesetzt wird, **dadurch gekennzeichnet, dass** R³ für -[CH₂-(CH(CH₃))-O]ₙ-[CH₂-(CH(CH₃))]-OH steht und n verschiedene Werte im Bereich von 8 bis 11 besitzt, besonders bevorzugt = 8, 9, 10 oder 11 ist.

5. Verfahren gemäß wenigstens einem der Ansprüche 1 bis 4, **dadurch gekennzeichnet dass** als Katalysator eine Mischung der tertiären Amine der Formel (I) eingesetzt wird, **dadurch gekennzeichnet, dass** die Katalysatoren verschiedene Reste R² mit geradkettigem oder verzweigtem C₁₆-, C₁₇-, C₁₈-, C₁₉-, C₂₀- oder C₂₁-Alkyl aufweisen.

6. Verfahren gemäß wenigstens einem der Ansprüche 1 bis 5, **dadurch gekennzeichnet, dass** Dialkyldicarbonate der Formel (II) 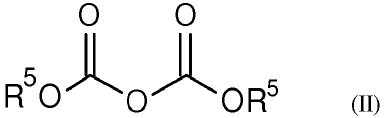 worin
R⁵ für geradkettiges oder verzweigtes C₁-C₂₀-Alkyl steht,
durch Umsetzung von Halogenameisensäurealkylester der Formel (III) 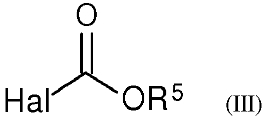
worin
Hal für Halogen, bevorzugt für F, Cl, Br, I, insbesondere für Chlor steht, und
R⁵ für geradkettiges oder verzweigtes C₁-C₂₀-Alkyl steht,
**dadurch gekennzeichnet, dass** die Umsetzung in Gegenwart mindestens eines tertiären Amins der Formel (I)
NR¹R²R³ (I)
worin
R¹ = geradkettiges oder verzweigtes C₁-C₆-Alkyl ist,
R² = geradkettiges oder verzweigtes C₁₆-C₂₂-Alkyl und
R³ = -[CH₂-R⁴-O]ₙ-[CH₂-R⁴]-OH ist, wobei n = 0 bis 12 und R⁴ = -[CH₂-CH_{2]}-, -[CH(CH₃)]- oder -CH₂- ist,
als Katalysator durchgeführt wird.

7. Verfahren gemäß wenigstens einem der Ansprüche 1 bis 4, **dadurch gekennzeichnet, dass** die Alkalihydroxide, Erdalkalihydroxide und/oder-carbonate in Form wässriger Lösungen einsetzt werden.

8. Verfahren gemäß wenigstens einem der Ansprüchen 1 bis 5, **dadurch gekennzeichnet, dass** man mindestens ein mit Wasser nicht mischbares organisches Lösungsmittel aus der Reihe der aliphatischen und aromatischen Kohlenwasserstoffe, der chlorierten Kohlenwasserstoffe, der Dialkylcarbonate oder der mit Wasser nicht mischbaren Ether und Ester einsetzt.

9. Verfahren gemäß wenigstens einem der Ansprüche 1 bis 6, **dadurch gekennzeichnet, dass** die Menge an eingesetztem tertiären Aminen der Formel (I) 0,001 bis 0,5 Mol-%, bezogen auf Halogenameisensäureester, beträgt.

10. Verfahren gemäß wenigstens einem der Ansprüche 1 bis 7, **dadurch gekennzeichnet, dass** die Reaktion bei einer Temperatur zwischen -10°C und der Siedetemperatur (bei Normaldruck) des eingesetzten Halogenameisensäureesters durchgeführt wird.

11. Verfahren gemäß wenigstens einem der Ansprüche 1 bis 8, **dadurch gekennzeichnet, dass** die Umsetzung in kontinuierlicher Arbeitsweise durchgeführt wird.

12. Verfahren gemäß wenigstens einem der Ansprüche 1 bis 9, **dadurch gekennzeichnet, dass** nach Beendigung der Umsetzung die Aufarbeitung des Reaktionsgemisches zur Abtrennung des Dialkylcarbonats durch Phasentrennung und anschließender mehrstufiger Destillation der organischen Phase erfolgt.

## Claims

1. Method for preparing dialkyl dicarbonates by reacting the corresponding alkyl haloformates with at least one alkali metal hydroxide, alkaline earth metal hydroxide and/or carbonate in the presence of at least one water-immiscible organic solvent and in the presence of a catalyst, **characterized in that** the catalyst used is at least one tertiary amine of the formula (I)
NR¹R²R³ (I)
where
R¹ = straight-chain or branched C₁-C₆-alkyl,
R² = straight-chain or branched C₁₆-C₂₂-alkyl and
R³ = -[CH₂-R⁴-O]ₙ-[CH₂-R⁴]-OH, where n = 0 to 12
and R⁴ = -[CH₂-CH₂]-, -[CH(CH₃)]- or -CH₂-.

2. Method according to Claim 1, **characterized in that** the catalyst used is at least one tertiary alkylamine of the formula (I) according to Claim 1, where R¹= methyl, ethyl, propyl or butyl and R² = C₁₇-C₂₀-alkyl and R³ = -[CH₂-CH(CH₃)-O]ₙ-[CH₂-CH(CH₃)]-OH or -[CH₂-CH₂-CH₂-O]ₙ-[CH₂-CH₂-CH₂]-OH where n = 6 to 10.

3. Method according to Claim 1 or 2, **characterized in that** the catalyst used is at least one tertiary amine of the formula (I) according to Claim 1, where R¹ = methyl, R² = straight-chain C₁₈-alkyl and R³ = -[CH₂-CH(CH₃)-O]ₙ-[CH₂-CH(CH₃)]-OH where n = 8.

4. Method according to at least one of Claims 1 to 3, **characterized in that** the catalyst used is a mixture of two or more tertiary amines of the formula (I), **characterized in that** R³ is -[CH₂-(CH(CH₃))-O]ₙ-[CH₂-(CH(CH₃))]-OH and n has various values in the range from 8 to 11, particularly preferably = 8, 9, 10 or 11.

5. Method according to at least one of Claims 1 to 4, **characterized in that** the catalyst used is a mixture of the tertiary amines of the formula (I), **characterized in that** the catalysts comprise various radicals R² that are straight-chain or branched C₁₆-, C₁₇-, C₁₈-, C₁₉-, C₂₀- or C₂₁-alkyl.

6. Method according to at least one of Claims 1 to 5, **characterized in that** dialkyl dicarbonates of the formula (II) 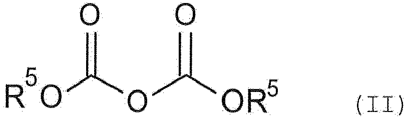 where
R⁵ is straight-chain or branched C₁-C₂₀-alkyl,
by reacting alkyl haloformates of the formula (III) 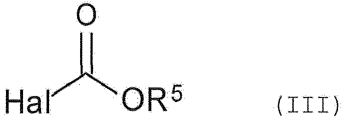
where
Hal is halogen, preferably F, Cl, Br, I, especially chlorine, and
R⁵ is straight-chain or branched C₁-C₂₀-alkyl,
**characterized in that** the reaction is carried out in the presence of at least one tertiary amine of the formula (I) as catalyst
NR¹R²R³ (I)
where
R¹ = straight-chain or branched C₁-C₆-alkyl,
R² = straight-chain or branched C₁₆-C₂₂-alkyl and
R³ = -[CH₂-R⁴-O]ₙ-[CH₂-R⁴]-OH, where n = 0 to 12 and R⁴ = -[CH₂-CH₂]-, -[CH(CH₃))- or -CH₂-.

7. Method according to at least one of Claims 1 to 4, **characterized in that** the alkali metal hydroxides, alkaline earth metal hydroxides and/or carbonates are used in the form of aqueous solutions.

8. Method according to at least one of Claims 1 to 5, **characterized in that** at least one water-immiscible organic solvent is used from the series of the aliphatic and aromatic hydrocarbons, the chlorinated hydrocarbons, the dialkyl carbonates or the water-immiscible ethers and esters.

9. Method according to at least one of Claims 1 to 6, **characterized in that** the amount of tertiary amines of the formula (I) used is 0.001 to 0.5 mol%, based on haloformic acid ester.

10. Method according to at least one of Claims 1 to 7, **characterized in that** the reaction is carried out at a temperature between -10°C and the boiling temperature (at standard pressure) of the haloformic acid ester used.

11. Method according to at least one of Claims 1 to 8, **characterized in that** the reaction is carried out in continuous operation.

12. Method according to at least one of Claims 1 to 9, **characterized in that**, after termination of the reaction, the reaction mixture is worked up to separate off the dialkyl carbonate by phase separation and subsequent multi-stage distillation of the organic phase.

## Revendications

1. Procédé de préparation de dicarbonates de dialkyle par conversion des esters alkyliques d'acides halogénoformiques correspondants avec au moins un hydroxyde alcalin, hydroxyde et/ou carbonate alcalino-terreux en présence d'au moins un solvant organique non miscible avec l'eau et en présence d'un catalyseur, **caractérisé en ce qu'**en tant que catalyseur, au moins une amine tertiaire de la formule (I) :
NR¹R²R³ (I)
dans laquelle
R¹ = alkyle en C₁-C₆ à chaîne linéaire ou ramifié,
R² = alkyle en C₁₆-C₂₂ à chaîne linéaire ou ramifié, et
R³ = -[CH₂-R⁴-O]ₙ-[CH₂-R⁴]-OH, où n = 0 à 12 et R⁴ = - [CH₂-CH₂]-, -[CH(CH₃)]- ou -CH₂-, est utilisée.

2. Procédé selon la revendication 1, **caractérisé en ce qu'**en tant que catalyseur, au moins une alkylamine tertiaire de la formule (I) selon la revendication 1 est utilisée, dans laquelle R¹ = méthyle, éthyle, propyle ou butyle, et R² = alkyle en C₁₇-C₂₀, et R³ = - [CH₂-CH(CH₃)-O]ₙ-[CH₂-CH(CH₃)]-OH ou -[CH₂-CH₂-CH₂-O]ₙ-[CH₂-CH₂-CH₂]-OH avec n = 6 à 10.

3. Procédé selon la revendication 1 ou 2, **caractérisé en ce qu'**en tant que catalyseur, au moins une amine tertiaire de la formule (I) selon la revendication 1 est utilisée, dans laquelle R¹ = méthyle, R² = alkyle en C₁₈ à chaîne linéaire, et R³ = -[CH₂-CH(CH₃)-O]ₙ-[CH₂-CH(CH₃)]-OH avec n = 8.

4. Procédé selon au moins l'une quelconque des revendications 1 à 3, **caractérisé en ce qu'**en tant que catalyseur, un mélange de deux ou davantage d'amines tertiaires de la formule (I) est utilisé, **caractérisé en ce que** R³ représente -[CH₂-(CH(CH₃))-O]ₙ-[CH₂-(CH(CH₃))]-OH et n présente différentes valeurs dans la plage allant de 8 à 11, de manière particulièrement préférée = 8, 9, 10 ou 11.

5. Procédé selon au moins l'une quelconque des revendications 1 à 4, **caractérisé en ce qu'**en tant que catalyseur, un mélange des amines tertiaires de la formule (I) est utilisé, **caractérisé en ce que** les catalyseurs comprennent différents radicaux R² contenant un alkyle en C₁₆, C₁₇, C₁₈, C₁₉, C₂₀ ou C₂₁ à chaîne linéaire ou ramifié.

6. Procédé selon au moins l'une quelconque des revendications 1 à 5, **caractérisé en ce que** des dicarbonates de dialkyle de la formule (II) : 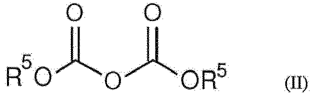 dans laquelle
R⁵ représente alkyle en C₁-C₂₀ à chaîne linéaire ou ramifié,
par conversion d'esters alkyliques d'acides halogénoformiques de la formule (III) : 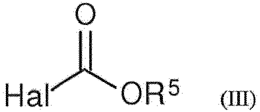
dans laquelle
Hal représente halogène, de préférence F, Cl, Br, I, notamment chlore, et
R⁵ représente alkyle en C₁-C₂₀ à chaîne linéaire ou ramifié,
**caractérisé en ce que** la conversion est réalisée en présence d'au moins une amine tertiaire de la formule (I) :
NR¹R²R³ (I)
dans laquelle
R¹ = alkyle en C₁-C₆ à chaîne linéaire ou ramifié,
R² = alkyle en C₁₆-C₂₂ à chaîne linéaire ou ramifié, et
R³ = -[CH₂-R⁴-O]ₙ-[CH₂-R⁴]-OH, où n = 0 à 12 et R⁴ = - [CH₂-CH₂]-, -[CH(CH₃)]- ou -CH₂-, en tant que catalyseur.

7. Procédé selon au moins l'une quelconque des revendications 1 à 4, **caractérisé en ce que** les hydroxydes alcalins, hydroxydes et/ou carbonates alcalino-terreux sont utilisés sous la forme de solutions aqueuses.

8. Procédé selon au moins l'une quelconque des revendications 1 à 5, **caractérisé en ce qu'**au moins un solvant organique non miscible avec l'eau de la série des hydrocarbures aliphatiques et aromatiques, des hydrocarbures chlorés, des carbonates de dialkyle ou des éthers et esters non miscibles avec l'eau est utilisé.

9. Procédé selon au moins l'une quelconque des revendications 1 à 6, **caractérisé en ce que** la quantité d'amines tertiaires de la formule (I) utilisées est de 0,001 à 0,5 % en moles, par rapport aux esters d'acides halogénoformiques.

10. Procédé selon au moins l'une quelconque des revendications 1 à 7, **caractérisé en ce que** la réaction est réalisée à une température comprise entre -10 °C et la température d'ébullition (à pression normale) de l'ester d'acide halogénoformique utilisé.

11. Procédé selon au moins l'une quelconque des revendications 1 à 8, **caractérisé en ce que** la conversion est réalisée en mode de travail continu.

12. Procédé selon au moins l'une quelconque des revendications 1 à 9, **caractérisé en ce qu'**après la fin de la conversion, le traitement du mélange réactionnel a lieu pour la séparation du carbonate de dialkyle par séparation de phases et ensuite distillation en plusieurs étapes de la phase organique.
